# EUROPEAN PATENT APPLICATION

(11) **EP 4 174 174 A1**
(43) Date of publication of application: **03.05.2023**
(21) Application number: 21382978.1
(22) Date of filing: 29.10.2021
(51) Int. Cl.: C12N 9/02, C12P 19/14

(54) **TAILOR-MADE EXTREMOPHILIC FUNGAL LACCASES**

(71) Applicant: Consejo Superior de Investigaciones Cientificas, 28006 Madrid (ES)
(72) Inventor: Rodríguez Escribano, David, 28040 Madrid (ES); Pliego Magán, Rocío, 28040 Madrid (ES); De Salas De la Cuadra, Felipe, 28040 Madrid (ES); Aza Toca, Pablo, 28040 Madrid (ES); Molpeceres García, Gonzalo, 28040 Madrid (SG); Camarero Fernández, Susana, 28040 Madrid (ES)
(74) Representative: Pons

(57) **Abstract**

The present invention relates to novel, tailor-made alkaliphilic and thermotolerant laccases developed by enzyme directed evolution for use in wood conversion, biomass biorefineries and green chemistry.

## Description

The present invention relates to novel, tailor-made alkaliphilic and thermotolerant laccases developed by enzyme directed evolution for use in wood conversion, biomass biorefineries and green chemistry.

### STATE OF THE ART

The exploitation of cellulose for paper pulp production involves the removal of lignin polymer that protects cellulose against microbial attack in the plant cell wall and gives structural support to the plant. During kraft pulping, the predominant technology for paper pulp production worldwide, wood lignin is solubilized under strong alkaline conditions. The resulting soluble extract has traditionally been burned to generate energy for the mill. However, lignin constitutes the main renewable source of aromatic compounds; thus, the current tendency is to valorise the industrial lignins into new bio-based products. Still, kraft lignins are scarcely exploited nowadays because there is no established technology for their valorisation. Biotechnology can contribute to this aim by providing enzymatic biocatalysts able to convert kraft lignins into new components and products.

Laccases are multi-copper oxidases secreted by white-rot fungi during wood decay for the degradation of lignin polymer. The ability of fungal laccases to depolymerize lignin, with preference for oxidizing lignin phenols, and to oxidize a range of aromatic compounds, using oxygen from the air as the unique requirement, make these enzymes biocatalysts of choice for application in different sectors.

Fungal laccases are particularly suited to oxidize and depolymerize kraft lignins due to the high content of phenolic units of these lignins. However, the alkaline pH (pH ≥ 10) at which kraft lignins are soluble hinders the utilization of wild-type fungal laccases that are only active at acid pH. Thus, there is a need to develop new enzymes with activity at high pH values which may be used in different industrial applications.

### DETAILED DESCRIPTION OF THE INVENTION

The present invention relates to a series of novel tailor-made alkaliphilic and thermotolerant laccases developed by enzyme directed evolution and suitable for highly demanding industrial operation conditions. The present inventors have identified a number of novel mutations that confer laccases unexpected extremophilic properties of particular interest for their exploitation as biocatalysts in different industrial applications, including but not limited to, applications in the pulp and paper sector. By way of nonlimiting example, the novel enzymes may be in used wood conversion for the valorisation of kraft lignins, delignification and bleaching of kraft pulps, and for wood fibre bonding and formulation of lignin-based resins in wood board manufacture.

Thus, in a first aspect, the invention relates to an isolated nucleic acid ("polynucleotide of the invention") encoding a polypeptide with thermotolerant laccase activity at neutral and alkaline pHs ("polypeptide of the invention"), wherein the amino acid sequence of the polypeptide encoded by the isolated nucleic acid: (i) has a sequence identity of at least 90% with SEQ ID NO: 1; and (ii) comprises (a) an amino acid mutation in the position homologous to position 392 of SEQ ID NO: 1; and (b) an isoleucine residue in the position homologous to position 396 of SEQ ID NO:1.

The term "nucleic acid" relates to any combination of nucleotide monomers which are connected to each other through covalent bonds. Within the present invention, the terms nucleic acid, nucleotide and polynucleotide are used interchangeably. The term "nucleotide sequence" relates to a polynucleotide sequence, and any variants, homologues, fragments and derivatives thereof. The nucleotide sequence may be DNA or RNA of genomic or synthetic or recombinant origin which may be double- or single-stranded, whether representing the sense or antisense strand. Preferably, the term "nucleotide sequence" means DNA. More preferably, the term "nucleotide sequence" means DNA prepared by use of recombinant DNA techniques (i.e., recombinant DNA).

The term "mutation" relates to any permanent changes in a nucleotide or polynucleotide sequence. There are three types of DNA mutations: base substitutions, deletions and insertions. Base substitutions are generally called point mutations, and they may be silent, missense or nonsense.

Silent point mutations do not result in any changes in the amino acid sequence of the polypeptide encoded by the nucleotide sequence. Nonsense point mutations result in a stop codon, truncating translation the polypeptide encoded by the nucleotide sequence and most likely leading to a non-functional protein.

Missense point mutations result in the generation of a codon that specifies a different amino acid, resulting in changes in the amino acid sequence of the polypeptide encoded by the nucleotide sequence, and hence leading to a different polypeptide sequence. Missense mutations may be either conservative or nonconservative; if the structure and properties of the substituted amino acid are very similar to the original amino acid, the mutation is said to be conservative and will most likely have little effect on the resultant protein's structure/function.

Within the present invention, the term "mutation" relates to missense point mutations. For the avoidance of any doubt, the missense point mutations in the nucleotide sequence of the polynucleotide of the invention lead to changes (mutations) in the amino acid sequence of the polypeptide of the invention; these changes are also referred to as "substitutions" throughout the present application.

The nucleotide sequence of the invention may be modified by introduction of nucleotide substitutions that do not result in a change in the amino acid sequence of the polypeptide, but which correspond to the codon usage of the host organism intended for production of the enzyme. In some embodiments of the invention, the nucleotide sequence of the invention is optimized for its expression in *Escherichia coli.* In some embodiments of the invention, the nucleotide sequence of the invention is optimized for its expression in *Pichia Pastoris.* In some embodiments of the invention, the nucleotide sequence of the invention is optimized for its expression in *Sacharomyces cerevisiae.*

In some embodiments of the invention, the nucleotide sequence of the invention is optimized for its expression in filamentous fungi. In some specific embodiments of the invention, the nucleotide sequence of the invention is optimized for its expression in *Aspergillus oryzae.*

The nucleotide sequence of the invention may comprise a signal peptide coding region that encodes a signal peptide linked to the N-terminus of a variant, wherein said signal peptide directs the variant into the cell's secretory pathway. The 5'-end of the coding sequence of the polynucleotide may inherently contain a signal peptide coding sequence naturally linked in translation reading frame with the segment of the coding sequence that encodes the polypeptide. Alternatively, the 5'-end of the coding sequence may contain a signal peptide coding sequence that is foreign to the coding sequence. A foreign signal peptide coding sequence may be required where the coding sequence does not naturally contain a signal peptide coding sequence. Alternatively, a foreign signal peptide coding sequence may simply replace the natural signal peptide coding sequence in order to enhance secretion of the variant. However, any signal peptide coding sequence that directs the expressed variant into the secretory pathway of a host cell may be used.

Effective signal peptide coding sequences for bacterial host cells include, without being limited to, signal peptide coding sequences obtained from the genes for *Bacillus* NCIB 11837 *maltogenic* amylase, *Bacillus licheniformis* subtilisin, *Bacillus licheniformis* beta-lactamase, *Bacillus stearothermophilus* alpha-amylase, *Bacillus stearothermophilus* neutral proteases (nprT, nprS, nprM), and *Bacillus subtilis* prsA.

Effective signal peptide coding sequences for yeast host cells include, without being limited to, those obtained or engineered from the secretion signal sequences (complete or fragments) of the genes of alpha mating factor, invertase, killer protein and alpha-galactosidase from *Saccharomyces cerevisiae.* Further information on host cells and signal peptide coding sequences for yeast host cells is provided elsewhere in this application.

As used herein, the term "polypeptide" relates to any biomolecule comprising any number of amino acid residues joined together by peptide bonds. Within the present invention, the terms polypeptide(s), peptide(s) and protein(s) are used interchangeably.

Polypeptides may contain amino acids other than the 20 gene-encoded amino acids. They may also include amino acid sequences modified either by natural processes, such as post-translational processing, or by chemical modification techniques which are well known in the art. Modifications can occur anywhere in a polypeptide, including the polypeptide backbone, the amino acid side-chains and the amino or carboxyl termini.

The same type of modification may be present in the same or varying degrees at several sites in a given polypeptide. A given polypeptide may contain many types of modifications.

The terms "amino acid" and "residue" are used interchangeably within the context of the present application.

The term "isolated" relates to any substance in a form or environment which does not occur in nature. The term "isolated polypeptide" and "isolated polynucleotide", as used herein, refers, respectively, to any polypeptide or polynucleotide that has been removed from its natural environment.

As used herein, the term "laccase" relates to any enzyme which catalyses oxidation reactions coupled to four-electron reduction of molecular oxygen to water. Thus, the polypeptide of the invention is any polypeptide capable of catalysing said oxidation reactions.

Laccases are widely distributed in higher plants and fungi. They belong to the multicopper oxidases superfamily, and oxidise phenols and aromatic amines, and other aromatic compounds and metal ions, participating in cross-linking of monomers (by the coupling of reactive radical intermediates) and degradation of polymers. Laccases act on both phenolic and nonphenolic lignin-related compounds (in the presence of redox mediators), as well as on highly recalcitrant environmental pollutants, making the enzymes particularly useful in different biotechnological applications for lignocellulosic biorefineries and green chemistry, for example in paper and pulp industries, textile industries, wastewater treatment, xenobiotic degradation and bioremediation, and drug synthesis.

Importantly, whilst most laccases of fungal origin are only active at acidic pHs, the novel polypeptides of the invention show laccase activity at neutral and alkaline pHs. Within the context of this invention, any pH lower than 7 is considered acidic, and any pH higher than 7 is considered alkaline. A pH of 7 is considered neutral e.g., pure water.

Additionally, most laccases of fungal origin are only active at temperatures ranging from 30 and 55°C. However, the novel polypeptides of the invention retain its laccase activity at temperatures between 73°C and 80°C (inclusive). Thus, within the context of the present invention, the term "polypeptide with thermotolerant laccase activity"/"thermotolerant laccase"/"thermotolerant polypeptide", and any other variants, relates to any polypeptide that retains its laccase activity at neutral and/or alkaline pH and at temperatures between 73°C and 80°C (inclusive).

The amino acid sequence of the polypeptide encoded by the isolated nucleic acid of the invention has a sequence identity of at least 90% with SEQ ID NO: 1 (RY2 laccase; De Salas et al. 2019 Green Chem. 21 5374-5385).

The term "identity" or "sequence identity" as used herein refers to the degree of similarity between two nucleotide or amino acid sequences obtained by aligning the two sequences, i.e., the proportion of identical nucleotide or amino acids between two compared nucleotide sequences or polypeptides/proteins along their full-length sequence. Depending on the number of common residues between the aligned sequences, a different degree of identity, expressed as a percentage, will be obtained. The degree of identity between two amino acids or nucleotides sequences may be determined by conventional methods, for example, by standard sequence alignment algorithms known in the state of the art, such as BLAST (Altschul S.F. et al. 1990 J Mol Biol. 215(3): 403-10). The BLAST programmes, for example, BLASTN, BLASTX, and T BLASTX, BLASTP and TBLASTN, are in the public domain at The National Center for Biotechonology Information (NCBI) website. The skilled person would understand that any mutations in the nucleotide sequence of genes that lead to conservative amino acid substitutions at non-critical positions for the functionality of the protein are evolutionarily neutral mutations which do not affect its global structure or its functionality.

Within the context of the present invention, any peptides, polypeptides or proteins with an identity of at least 90% with SEQ ID NO: 1 are functionally equivalent variants of SEQ ID NO: 1.

The term "functionally equivalent variant" refers to a polypeptide comprising one or more alterations, such as substitutions, insertions, deletions and/or truncations, of one or more specific amino acid residues at one or more specific positions in the protein, said polypeptide having the same function as the original protein. Functionally equivalent variants include polypeptide fragments. The term "fragment" as used herein relates to a polypeptide or a domain thereof having one or more (e.g., several) amino acids absent from the amino and/or carboxyl terminus of the amino acid sequence, wherein the fragment has the same activity as the whole protein.

Functionally equivalent variants of SEQ ID NO: 1 maintain the same properties as the sequence to which they refer, i.e., they show laccase activity. Different methods for assessing whether a given peptide, polypeptide or protein is a functionally equivalent variant of the sequence SEQ ID NO: 1 may be found in the examples section of the present description.

In some embodiments, the polypeptide of the invention has an identity of at least 91; 92; 93; 94; 95; 96; 97; 98; or 99% with amino acid sequence SEQ ID NO: 1.

The amino acid sequence of the polypeptide encoded by the nucleic acid of the invention comprises an amino acid mutation in the position homologous to position 392 of SEQ ID NO:1. In some embodiments, in the amino acid sequence of the polypeptide encoded by the isolated nucleic acid of the invention, the amino acid in the position homologous to position 392 of SEQ ID NO: 1 is substituted by an amino acid selected from the group consisting of: serine; glutamine; asparagine; and threonine. In some specific embodiments, the phenylalanine residue in position 392 of SEQ ID NO: 1 is substituted by: serine ("F392S"); glutamine ("F392Q"); asparagine ("F392N"); or threonine ("F392T").

In some embodiments of the invention, the amino acid in the position homologous to position 392 of SEQ ID NO: 1 is substituted by serine. In some specific embodiments, the phenylalanine residue in position 392 of SEQ ID NO: 1 is substituted by serine ("F392S").

In some embodiments of the invention, the amino acid in the position homologous to position 392 of SEQ ID NO: 1 is substituted by glutamine. In some specific embodiments, the phenylalanine residue in position 392 of SEQ ID NO: 1 is substituted by glutamine ("F392Q").

In some embodiments of the invention, the amino acid in the position homologous to position 392 of SEQ ID NO: 1 is substituted by asparagine. In some specific embodiments, the phenylalanine residue in position 392 of SEQ ID NO: 1 is substituted by asparagine ("F392N").

In some embodiments of the invention, the amino acid in the position homologous to position 392 of SEQ ID NO: 1 is substituted by threonine. In some specific embodiments, the phenylalanine residue in position 392 of SEQ ID NO: 1 is substituted by threonine ("F392T").

The amino acid sequence of the polypeptide encoded by the nucleic acid of the invention comprises an isoleucine residue in the position homologous to position 396 of SEQ ID NO: 1. Thus, in some specific embodiments, the phenylalanine residue in position 396 of SEQ ID NO: 1 is substituted by isoleucine ("F396I").

Variations in the position homologous to position 396 of SEQ ID NO: 1 have been previously described (ES2525195 B1). Specifically, mutation F396I has been described as responsible for the improvement of laccase activity at neutral pH.

However, the specific effect of amino acid substitutions in the position homologous to position 392 of SEQ ID NO: 1 has been characterized herein for the first time. As shown by the present inventors, the residue in the position homologous to position 392 of SEQ ID NO: 1 is essential for tuning the pH-dependent enzyme activity. Indeed, the substitution of the residue in said position by a polar uncharged residue, in combination with the presence of an isoleucine residue in the position homologous to position 396 of SEQ ID NO: 1, further shifts the laccase optimum pH from pH 5 to pH 6 and enhances the activity at pH 7 and pH 8.

Particularly, the presence of a polar uncharged residue (e.g., asparagine, threonine) at position homologous 392 of SEQ ID NO: 1 has a clear, positive effect on the activity of the polypeptides of the invention at neutral and basic pHs.

In some specific embodiments, the nucleic acid of the invention comprises, or consists of, a nucleic acid sequence that has a sequence identity of at least 60% with at least one sequence selected from the group consisting of: SEQ ID NO: 27 to 42; SEQ ID NO: 45 to 50; and SEQ ID NO: 52.

In some specific embodiments, the amino acid sequence of the polypeptide encoded by the nucleic acid of the invention comprises, or consists of, an amino acid sequence that has a sequence identity of at least 90% with at least one sequence selected from the group consisting of: SEQ ID NO: 2 to 17; SEQ ID NO: 20 to 25; and SEQ ID NO: 51.

In some embodiments of the invention, the amino acid sequence of the polypeptide encoded by the isolated nucleic acid of the invention further comprises an amino acid mutation in the position homologous to position 460 of SEQ ID NO: 1.

Thus, in some embodiments, the amino acid sequence of the polypeptide of the invention comprises: (i) an amino acid mutation in the position homologous to position 392 of SEQ ID NO: 1; (ii) an isoleucine residue in the position homologous to position 396 of SEQ ID NO: 1; and (iii) an amino acid mutation in the position homologous to position 460 of SEQ ID NO: 1.

In some specific embodiments, the amino acid sequence of the polypeptide of the invention comprises: (i) a residue selected from the group consisting of: serine; glutamine; asparagine; and threonine in the position homologous to position 392 of SEQ ID NO: 1; (ii) an isoleucine residue in the position homologous to position 396 of SEQ ID NO: 1; and (iii) an amino acid mutation in the position homologous to position 460 of SEQ ID NO: 1.

In some embodiments of the invention, the amino acid in the position homologous to position 460 of SEQ ID NO: 1 is substituted by methionine. In some specific embodiments, the phenylalanine residue in position 460 of SEQ ID NO: 1 is substituted by methionine ("F460M").

Thus, in some specific embodiments, the amino acid sequence of the polypeptide of the invention comprises: (i) a residue selected from the group consisting of: serine; glutamine; asparagine; tyrosine; or threonine in the position homologous to position 392 of SEQ ID NO: 1; (ii) an isoleucine residue in the position homologous to position 396 of SEQ ID NO: 1; and (iii) a methionine residue in the position homologous to position 460 of SEQ ID NO: 1.

In some specific embodiments, the amino acid sequence of the polypeptide of the invention comprises: (i) an asparagine residue in the position homologous to position 392 of SEQ ID NO: 1; (ii) an isoleucine residue in the position homologous to position 396 of SEQ ID NO: 1; and (iii) a methionine residue in the position homologous to position 460 of SEQ ID NO: 1.

As shown herein, mutations in the position homologous to position 460 of SEQ ID NO: 1 produce a clear shift in laccase optimum pH from pH 6 to pH 8. Indeed, introduction of the mutation F460M has allowed the present inventors to measure the kinetic parameters of the enzyme at pH 9 for the first time.

In some specific embodiments, the nucleic acid of the invention comprises, or consists of, a nucleic acid sequence that has a sequence identity of at least 60% with at least one sequence selected from the group consisting of: SEQ ID NO: 28 to 31; SEQ ID NO: 33 to 42; and SEQ ID NO: 45 to 50.

In some specific embodiments, the amino acid sequence of the polypeptide encoded by the nucleic acid of the invention comprises, or consists of, an amino acid sequence that has a sequence identity of at least 90% with at least one sequence selected from the group consisting of: SEQ ID NO: 3 to 6; SEQ ID NO: 8 to 17; and SEQ ID NO: 20 to 25.

In some embodiments of the invention, the amino acid sequence of the polypeptide encoded by the isolated nucleic acid of the invention further comprises an amino acid mutation in the position homologous to position 263 of SEQ ID NO: 1.

Thus, in some embodiments, the amino acid sequence of the polypeptide of the invention comprises: (i) an amino acid mutation in the position homologous to position 392 of SEQ ID NO: 1; (ii) an isoleucine residue in the position homologous to position 396 of SEQ ID NO: 1; and (iii) an amino acid mutation in the position homologous to position 263 of SEQ ID NO: 1.

In some specific embodiments, the amino acid sequence of the polypeptide of the invention comprises: (i) a residue selected from the group consisting of: serine; glutamine; asparagine and threonine in the position homologous to position 392 of SEQ ID NO: 1; (ii) an isoleucine residue in the position homologous to position 396 of SEQ ID NO: 1;1; (iii) an amino acid mutation in the position homologous to position 263 of SEQ ID NO: 1.

In some embodiments of the invention, the amino acid in the position homologous to position 263 of SEQ ID NO: 1 is substituted by an amino acid selected from the group consisting of: valine; alanine; and glycine. In some specific embodiments, the aspartic acid residue in position 263 of SEQ ID NO: 1 is substituted by: valine ("D263V"); alanine ("D263A"); or glycine ("D263G").

In some embodiments of the invention, the amino acid in the position homologous to position 263 of SEQ ID NO: 1 is substituted by valine. In some specific embodiments, the aspartic acid residue in position 263 of SEQ ID NO: 1 is substituted by valine ("D263V").

Thus, in some embodiments, the amino acid sequence of the polypeptide of the invention comprises: (i) a residue selected from the group consisting of: serine; glutamine; asparagine; and threonine in the position homologous to position 392 of SEQ ID NO: 1; (ii) an isoleucine residue in the position homologous to position 396 of SEQ ID NO: 1; and (iii) a residue selected from the group consisting of: valine; alanine; and glycine in the position homologous to position 263 of SEQ ID NO: 1.

In some specific embodiments, the amino acid sequence of the polypeptide of the invention comprises: (i) a residue selected from the group consisting of: serine; glutamine; asparagine; and threonine in the position homologous to position 392 of SEQ ID NO: 1; (ii) an isoleucine residue in the position homologous to position 396 of SEQ ID NO: 1; and (iii) a valine residue in the position homologous to position 263 of SEQ ID NO: 1.

In some specific embodiments, the amino acid sequence of the polypeptide of the invention comprises: (i) an asparagine residue in the position homologous to position 392 of SEQ ID NO: 1; (ii) an isoleucine residue in the position homologous to position 396 of SEQ ID NO: 1; and (iii) a valine residue in the position homologous to position 263 of SEQ ID NO: 1.

In some embodiments, the amino acid sequence of the polypeptide of the invention comprises: (i) an amino acid mutation in the position homologous to position 392 of SEQ ID NO: 1; (ii) an isoleucine residue in the position homologous to position 396 of SEQ ID NO: 1; (iii) an amino acid mutation in the position homologous to position 460 of SEQ ID NO: 1; and (iv) an amino acid mutation in the position homologous to position 263 of SEQ ID NO: 1.

In some specific embodiments, the amino acid sequence of the polypeptide of the invention comprises: (i) a residue selected from the group consisting of: serine; glutamine; asparagine and threonine in the position homologous to position 392 of SEQ ID NO: 1; (ii) an isoleucine residue in the position homologous to position 396 of SEQ ID NO: 1; (iii) a methionine residue in the position homologous to position 460 of SEQ ID NO: 1; and (iv) a residue selected from the group consisting of: valine; alanine; and glycine in the position homologous to position 263 of SEQ ID NO: 1.

In some specific embodiments, the amino acid sequence of the polypeptide of the invention comprises: (i) a residue selected from the group consisting of: serine; glutamine; asparagine; and threonine in the position homologous to position 392 of SEQ ID NO: 1; (ii) an isoleucine residue in the position homologous to position 396 of SEQ ID NO: 1; (iii) a methionine residue in the position homologous to position 460 of SEQ ID NO: 1; and (iv) a valine residue in the position homologous to position 263 of SEQ ID NO: 1.

In some specific embodiments, the amino acid sequence of the polypeptide of the invention comprises: (i) an asparagine residue in the position homologous to position 392 of SEQ ID NO: 1; (ii) an isoleucine residue in the position homologous to position 396 of SEQ ID NO: 1; (iii) a methionine residue in the position homologous to position 460 of SEQ ID NO: 1; and (iv) a valine residue in the position homologous to position 263 of SEQ ID NO: 1.

Mutations in the position homologous to position 263 of SEQ ID NO: 1 substantially improve the catalytic efficiency of the enzyme at pH 8-10, as shown herein. Particularly, the presence of a small, non-charged, and non-polar residue at said position has a clear, positive effect on the activity of the polypeptides of the invention at neutral and basic pHs.

In some specific embodiments, the nucleic acid of the invention comprises, or consists of, a nucleic acid sequence that has a sequence identity of at least 60% with at least one sequence selected from the group consisting of: SEQ ID NO: 29 to 31; SEQ ID NO: 34 to 42; and SEQ ID NO: 45 to 50.

In some specific embodiments, the amino acid sequence of the polypeptide encoded by the nucleic acid of the invention comprises, or consists of, an amino acid sequence that has a sequence identity of at least 90% with at least one sequence selected from the group consisting of: SEQ ID NO: 4 to 6; SEQ ID NO: 9 to 17; and SEQ ID NO: 20 to 25.

In some embodiments of the invention, the amino acid sequence of the polypeptide encoded by the isolated nucleic acid of the invention further comprises an amino acid mutation in the position homologous to position 458 of SEQ ID NO:1.

Thus, in some embodiments, the amino acid sequence of the polypeptide of the invention comprises: (i) an amino acid mutation in the position homologous to position 392 of SEQ ID NO: 1; (ii) an isoleucine residue in the position homologous to position 396 of SEQ ID NO: 1; and (iii) an amino acid mutation in the position homologous to position 458 of SEQ ID NO: 1.

In some specific embodiments, the amino acid sequence of the polypeptide of the invention comprises: (i) a residue selected from the group consisting of: serine; glutamine; asparagine and threonine in the position homologous to position 392 of SEQ ID NO: 1; (ii) an isoleucine residue in the position homologous to position 396 of SEQ ID NO: 1; and (iii) an amino acid mutation in the position homologous to position 458 of SEQ ID NO: 1.

In some embodiments of the invention, the amino acid in the position homologous to position 458 of SEQ ID NO: 1 is substituted by an amino acid consisting of leucine; and threonine. In some specific embodiments, the alanine residue in position 458 of SEQ ID NO: 1 is substituted by: leucine ("A458L"); or threonine ("A458T").

Thus, in some specific embodiments, the amino acid sequence of the polypeptide of the invention comprises: (i) a residue selected from the group consisting of: serine; glutamine; asparagine and threonine in the position homologous to position 392 of SEQ ID NO: 1; (ii) an isoleucine residue in the position homologous to position 396 of SEQ ID NO: 1; and (iii) a residue selected from the group consisting of: leucine; and threonine in the position homologous to position 458 of SEQ ID NO: 1.

In some specific embodiments, the amino acid sequence of the polypeptide of the invention comprises: (i) a residue selected from the group consisting of: serine; glutamine; asparagine and threonine in the position homologous to position 392 of SEQ ID NO: 1; (ii) an isoleucine residue in the position homologous to position 396 of SEQ ID NO: 1; and (iii) a threonine residue in the position homologous to position 458 of SEQ ID NO: 1.

In some specific embodiments, the amino acid sequence of the polypeptide of the invention comprises: (i) an asparagine residue in the position homologous to position 392 of SEQ ID NO: 1; (ii) an isoleucine residue in the position homologous to position 396 of SEQ ID NO: 1; and (iii) a threonine residue in the position homologous to position 458 of SEQ ID NO: 1.

In some specific embodiments, the amino acid sequence of the polypeptide of the invention comprises: (i) a residue selected from the group consisting of: serine; glutamine; asparagine; and threonine in the position homologous to position 392 of SEQ ID NO: 1; (ii) an isoleucine residue in the position homologous to position 396 of SEQ ID NO: 1; and (iii) a leucine residue in the position homologous to position 458 of SEQ ID NO: 1.

In some specific embodiments, the amino acid sequence of the polypeptide of the invention comprises: (i) an asparagine residue in the position homologous to position 392 of SEQ ID NO: 1; (ii) an isoleucine residue in the position homologous to position 396 of SEQ ID NO: 1; and (iii) a leucine residue in the position homologous to position 458 of SEQ ID NO: 1.

In some embodiments, the amino acid sequence of the polypeptide of the invention comprises: (i) an amino acid mutation in the position homologous to position 392 of SEQ ID NO: 1; (ii) an isoleucine residue in the position homologous to position 396 of SEQ ID NO: 1; (iii) an amino acid mutation in the position homologous to position 460 of SEQ ID NO: 1; (iv) an amino acid mutation in the position homologous to position 263 of SEQ ID NO: 1 and (v) an amino acid mutation in the position homologous to position 458 of SEQ ID NO: 1 .

In some specific embodiments, the amino acid sequence of the polypeptide of the invention comprises: (i) a residue selected from the group consisting of: serine; glutamine; asparagine; and threonine in the position homologous to position 392 of SEQ ID NO: 1; (ii) an isoleucine residue in the position homologous to position 396 of SEQ ID NO: 1; (iii) a methionine residue in the position homologous to position 460 of SEQ ID NO: 1; (iv) a residue selected from the group consisting of: valine; alanine; and glycine in the position homologous to position 263 of SEQ ID NO: 1; and (v) a residue selected from the group consisting of: leucine; and threonine in the position homologous to position 458 of SEQ ID NO: 1.

In some specific embodiments, the amino acid sequence of the polypeptide of the invention comprises: (i) a residue selected from the group consisting of: serine; glutamine; asparagine; and threonine in the position homologous to position 392 of SEQ ID NO: 1; (ii) an isoleucine residue in the position homologous to position 396 of SEQ ID NO: 1; (iii) a methionine residue in the position homologous to position 460 of SEQ ID NO: 1; (iv) a valine residue in the position homologous to position 263 of SEQ ID NO: 1; and (v) a threonine residue in the position homologous to position 458 of SEQ ID NO: 1.

In some specific embodiments, the amino acid sequence of the polypeptide of the invention comprises: (i) an asparagine residue in the position homologous to position 392 of SEQ ID NO: 1; (ii) an isoleucine residue in the position homologous to position 396 of SEQ ID NO: 1; (iii) a methionine residue in the position homologous to position 460 of SEQ ID NO: 1; (iv) a valine residue in the position homologous to position 263 of SEQ ID NO: 1; and (v) a threonine residue in the position homologous to position 458 of SEQ ID NO: 1.

In some specific embodiments, the amino acid sequence of the polypeptide of the invention comprises: (i) a residue selected from the group consisting of: serine; glutamine; asparagine; and threonine in the position homologous to position 392 of SEQ ID NO: 1; (ii) an isoleucine residue in the position homologous to position 396 of SEQ ID NO: 1; (iii) a methionine residue in the position homologous to position 460 of SEQ ID NO: 1; (iv) a valine residue in the position homologous to position 263 of SEQ ID NO: 1; and (v) a leucine residue in the position homologous to position 458 of SEQ ID NO: 1.

In some specific embodiments, the amino acid sequence of the polypeptide of the invention comprises: (i) an asparagine residue in the position homologous to position 392 of SEQ ID NO: 1; (ii) an isoleucine residue in the position homologous to position 396 of SEQ ID NO: 1; (iii) a methionine residue in the position homologous to position 460 of SEQ ID NO: 1; (iv) a valine residue in the position homologous to position 263 of SEQ ID NO: 1; and (v) a leucine residue in the position homologous to position 458 of SEQ ID NO: 1.

In some specific embodiments, the nucleic acid of the invention comprises, or consists of, a nucleic acid sequence that has a sequence identity of at least 60% with at least one sequence selected from the group consisting of: SEQ ID NO: 30; 31; 35; 36; 38; 39; 41 42; 46; 47; 49; 50; and 52.

In some specific embodiments, the amino acid sequence of the polypeptide encoded by the nucleic acid of the invention comprises, or consists of, an amino acid sequence that has a sequence identity of at least 90% with at least one sequence selected from the group consisting of: SEQ ID NO: 5; 6; 10; 11; 13; 14; 16; 17; 21; 22; 24; 25; and 51.

Mutation of the residues in those positions homologous to position 458 of SEQ ID NO: 1 has a strong effect in the alkaliphilic activity of the enzyme. As shown herein, mutations A458L and A458T produce a remarkable shift of the laccase optimum pH. Furthermore, mutation A458L produces a notable increase in laccase catalytic efficiency for the oxidation of DMP at pH 10.

In some specific embodiments of the invention wherein the amino acid sequence of the polypeptide encoded by the polynucleotide of the invention comprises: (v) a residue selected from the group consisting of: leucine; and threonine in the position homologous to position 458 of SEQ ID NO: 1, the amino acid sequence of the polypeptide encoded by the polynucleotide of the invention further comprises an amino acid mutation in the position homologous to position 456 of SEQ ID NO: 1.

In said specific embodiments, the amino acid sequence of the polypeptide encoded by the polynucleotide of the invention comprises a methionine residue in the position homologous to position 456 of SEQ ID NO: 1. In some specific embodiments, the leucine residue in position 456 of SEQ ID NO: 1 is substituted by methionine ("L456M").

Mutation of the residue in the position homologous to position 456 of SEQ ID NO: 1 also has a strong effect in the alkaliphilic activity of the enzyme, particularly in combination with mutations in the position homologous to position 458 of SEQ ID NO: 1.

In some specific embodiments, the nucleic acid of the invention comprises, or consists of, a nucleic acid sequence that has a sequence identity of at least 60% with SEQ ID NO: 52.

In some specific embodiments, the amino acid sequence of the polypeptide encoded by the nucleic acid of the invention comprises, or consists of, an amino acid sequence that has a sequence identity of at least 90% with SEQ ID NO: 51.

In some embodiments of the invention, the amino acid sequence of the polypeptide encoded by the isolated nucleic acid of the invention further comprises amino acid mutations in the positions homologous to: position 424; 429; and 441 of SEQ ID NO: 1.

Thus, in some embodiments, the amino acid sequence of the polypeptide of the invention comprises: (i) an amino acid mutation in the position homologous to position 392 of SEQ ID NO: 1; (ii) an amino acid mutation in the position homologous to position 396 of SEQ ID NO: 1; and (iii) amino acid mutations in the positions homologous to: position 424; 429; and 441 of SEQ ID NO: 1.

In some specific embodiments, the amino acid sequence of the polypeptide of the invention comprises: (i) a residue selected from the group consisting of: serine; glutamine; asparagine; and threonine in the position homologous to position 392 of SEQ ID NO: 1; (ii) an isoleucine residue in the position homologous to position 396 of SEQ ID NO: 1; and (iii) amino acid mutations in the positions homologous to: position 424; 429; and 441 of SEQ ID NO: 1.

In some embodiments, the residue in the position homologous to position 424 of SEQ ID NO: 1 is substituted by a threonine residue. In some specific embodiments, the valine residue in position 424 of SEQ ID NO: 1 is substituted by threonine ("V424T").

In some embodiments, the residue in the position homologous to position 429 of SEQ ID NO: 1 is substituted by a threonine residue. In some specific embodiments, the serine residue in position 429 of SEQ ID NO: 1 is substituted by threonine ("S429T").

In some embodiments, the residue in the position homologous to position 441 of SEQ ID NO: 1 is substituted by an asparagine residue. In some specific embodiments, the aspartic residue in position 441 of SEQ ID NO: 1 is substituted by asparagine ("D441N").

Thus, in some specific embodiments, the amino acid sequence of the polypeptide of the invention comprises: (i) a residue selected from the group consisting of: serine; glutamine; asparagine; and threonine in the position homologous to position 392 of SEQ ID NO: 1; (ii) an isoleucine residue in the position homologous to position 396 of SEQ ID NO: 1; (iii) a threonine residue in the position homologous to position 424 of SEQ ID NO: 1; (iv) a threonine residue in the position homologous to position 429 of SEQ ID NO: 1; and (v) an asparagine residue in the position homologous to position 441 of SEQ ID NO: 1.

In some specific embodiments, the amino acid sequence of the polypeptide of the invention comprises: (i) an asparagine residue in the position homologous to position 392 of SEQ ID NO: 1; (ii) an isoleucine residue in the position homologous to position 396 of SEQ ID NO: 1; (iii) a threonine residue in the position homologous to position 424 of SEQ ID NO: 1; (iv) a threonine residue in the position homologous to position 429 of SEQ ID NO: 1; and (v) an asparagine residue in the position homologous to position 441 of SEQ ID NO: 1.

In some embodiments, the amino acid sequence of the polypeptide of the invention comprises: (i) an amino acid mutation in the position homologous to position 392 of SEQ ID NO: 1; (ii) an amino acid mutation in the position homologous to position 396 of SEQ ID NO: 1; (iii) an amino acid mutation in the position homologous to position 460 of SEQ ID NO: 1; and (v) amino acid mutations in the positions homologous to: position 424, 429 and 441 of SEQ ID NO: 1.

In some specific embodiments, the amino acid sequence of the polypeptide of the invention comprises: (i) a residue selected from the group consisting of: serine; glutamine; asparagine; and threonine in the position homologous to position 392 of SEQ ID NO: 1; (ii) an isoleucine residue in the position homologous to position 396 of SEQ ID NO: 1; (iii) a methionine residue in the position homologous to position 460 of SEQ ID NO: 1; (iv) a threonine residue in the position homologous to position 424 of SEQ ID NO: 1; (v) a threonine residue in the position homologous to position 429 of SEQ ID NO: 1; and (vi) an asparagine residue in the position homologous to position 441 of SEQ ID NO: 1.

In some specific embodiments, the amino acid sequence of the polypeptide of the invention comprises: (i) an asparagine residue in the position homologous to position 392 of SEQ ID NO: 1; (ii) an isoleucine residue in the position homologous to position 396 of SEQ ID NO: 1; (iii) a methionine residue in the position homologous to position 460 of SEQ ID NO: 1; (iv) a threonine residue in the position homologous to position 424 of SEQ ID NO: 1; (v) a threonine residue in the position homologous to position 429 of SEQ ID NO: 1; and (vi) an asparagine residue in the position homologous to position 441 of SEQ ID NO: 1.

In some embodiments, the amino acid sequence of the polypeptide of the invention comprises: (i) an amino acid mutation in the position homologous to position 392 of SEQ ID NO: 1; (ii) an isoleucine residue in the position homologous to position 396 of SEQ ID NO: 1; (iii) an amino acid mutation in the position homologous to position 460 of SEQ ID NO: 1; (iv) an amino acid mutation in the position homologous to position 263 of SEQ ID NO: 1; and (v) amino acid mutations in the positions homologous to: position 424; 429; and 441 of SEQ ID NO: 1.

In some specific embodiments, the amino acid sequence of the polypeptide of the invention comprises: (i) a residue selected from the group consisting of: serine; glutamine; asparagine; and threonine in the position homologous to position 392 of SEQ ID NO: 1; (ii) an isoleucine residue in the position homologous to position 396 of SEQ ID NO: 1; (iii) a methionine residue in the position homologous to position 460 of SEQ ID NO: 1; (iv) a residue selected from the group consisting of: valine; alanine; and glycine in the position homologous to position 263 of SEQ ID NO: 1; (v) a threonine residue in the position homologous to position 424 of SEQ ID NO: 1; (vi) a threonine residue in the position homologous to position 429 of SEQ ID NO: 1; and (vii) an asparagine residue in the position homologous to position 441 of SEQ ID NO: 1.

In some specific embodiments, the amino acid sequence of the polypeptide of the invention comprises: (i) a residue selected from the group consisting of: serine; glutamine; asparagine; and threonine in the position homologous to position 392 of SEQ ID NO: 1; (ii) an isoleucine residue in the position homologous to position 396 of SEQ ID NO: 1; (iii) a methionine residue in the position homologous to position 460 of SEQ ID NO: 1; (iv) a valine residue in the position homologous to position 263 of SEQ ID NO: 1; (v) a threonine residue in the position homologous to position 424 of SEQ ID NO: 1; (vi) a threonine residue in the position homologous to position 429 of SEQ ID NO: 1; and (vii) an asparagine residue in the position homologous to position 441 of SEQ ID NO: 1.

In some specific embodiments, the amino acid sequence of the polypeptide of the invention comprises: (i) an asparagine residue in the position homologous to position 392 of SEQ ID NO: 1; (ii) an isoleucine residue in the position homologous to position 396 of SEQ ID NO: 1; (iii) a methionine residue in the position homologous to position 460 of SEQ ID NO: 1; (iv) a valine residue in the position homologous to position 263 of SEQ ID NO: 1; (v) a threonine residue in the position homologous to position 424 of SEQ ID NO: 1; (vi) a threonine residue in the position homologous to position 429 of SEQ ID NO: 1; and (vii) an asparagine residue in the position homologous to position 441 of SEQ ID NO: 1.

In some embodiments, the amino acid sequence of the polypeptide of the invention comprises: (i) an amino acid mutation in the position homologous to position 392 of SEQ ID NO: 1; (ii) an amino acid mutation in the position homologous to position 396 of SEQ ID NO: 1; (iii) an amino acid mutation in the position homologous to position 460 of SEQ ID NO: 1; (iv) an amino acid mutation in the position homologous to position 263 of SEQ ID NO: 1; (v) an amino acid mutation in the position homologous to position 458 of SEQ ID NO: 1; and (vi) amino acid mutations in the positions homologous to: position 424; 429; and 441 of SEQ ID NO: 1.

In some specific embodiments, the amino acid sequence of the polypeptide of the invention comprises: (i) a residue selected from the group consisting of: serine; glutamine; asparagine; and threonine in the position homologous to position 392 of SEQ ID NO: 1; (ii) an isoleucine residue in the position homologous to position 396 of SEQ ID NO: 1; (iii) a methionine residue in the position homologous to position 460 of SEQ ID NO: 1; (iv) a residue selected from the group consisting of: valine; alanine; and glycine in the position homologous to position 263 of SEQ ID NO: 1; (v) a residue selected from the group consisting of: leucine; and threonine in the position homologous to position 458 of SEQ ID NO: 1; (vi) a threonine residue in the position homologous to position 424 of SEQ ID NO: 1; (vii) a threonine residue in the position homologous to position 429 of SEQ ID NO: 1; and (viii) an asparagine residue in the position homologous to position 441 of SEQ ID NO: 1.

In some specific embodiments, the amino acid sequence of the polypeptide of the invention comprises: (i) a residue selected from the group consisting of: serine; glutamine; asparagine; and threonine in the position homologous to position 392 of SEQ ID NO: 1; (ii) an isoleucine residue in the position homologous to position 396 of SEQ ID NO: 1; (iii) a methionine residue in the position homologous to position 460 of SEQ ID NO: 1; (iv) a valine residue in the position homologous to position 263 of SEQ ID NO: 1; (v) a threonine residue in the position homologous to position 458 of SEQ ID NO: 1; (vi) a threonine residue in the position homologous to position 424 of SEQ ID NO: 1; (vii) a threonine residue in the position homologous to position 429 of SEQ ID NO: 1; and (viii) an asparagine residue in the position homologous to position 441 of SEQ ID NO: 1.

In some specific embodiments, the amino acid sequence of the polypeptide of the invention comprises: (i) a residue selected from the group consisting of: serine; glutamine; asparagine; and threonine in the position homologous to position 392 of SEQ ID NO: 1; (ii) an isoleucine residue in the position homologous to position 396 of SEQ ID NO: 1; (iii) a methionine residue in the position homologous to position 460 of SEQ ID NO: 1; (iv) a valine residue in the position homologous to position 263 of SEQ ID NO: 1; (v) a leucine residue in the position homologous to position 458 of SEQ ID NO: 1; (vi) a threonine residue in the position homologous to position 424 of SEQ ID NO: 1; (vii) a threonine residue in the position homologous to position 429 of SEQ ID NO: 1; and (viii) an asparagine residue in the position homologous to position 441 of SEQ ID NO: 1.

In some specific embodiments, the amino acid sequence of the polypeptide of the invention comprises: (i) an asparagine residue in the position homologous to position 392 of SEQ ID NO: 1; (ii) an isoleucine residue in the position homologous to position 396 of SEQ ID NO: 1; (iii) a methionine residue in the position homologous to position 460 of SEQ ID NO: 1; (iv) a valine residue in the position homologous to position 263 of SEQ ID NO: 1; (v) a threonine residue in the position homologous to position 458 of SEQ ID NO: 1; (vi) a threonine residue in the position homologous to position 424 of SEQ ID NO: 1; (vii) a threonine residue in the position homologous to position 429 of SEQ ID NO: 1; and (viii) an asparagine residue in the position homologous to position 441 of SEQ ID NO: 1.

In some specific embodiments, the amino acid sequence of the polypeptide of the invention comprises: (i) an asparagine residue in the position homologous to position 392 of SEQ ID NO: 1; (ii) an isoleucine residue in the position homologous to position 396 of SEQ ID NO: 1; (iii) a methionine residue in the position homologous to position 460 of SEQ ID NO: 1; (iv) a valine residue in the position homologous to position 263 of SEQ ID NO: 1; (v) a leucine residue in the position homologous to position 458 of SEQ ID NO: 1; (vi) a threonine residue in the position homologous to position 424 of SEQ ID NO: 1; (vii) a threonine residue in the position homologous to position 429 of SEQ ID NO: 1; and (viii) an asparagine residue in the position homologous to position 441 of SEQ ID NO: 1.

In some specific embodiments, the nucleic acid of the invention comprises, or consists of, a nucleic acid sequence that has a sequence identity of at least 60% with at least one sequence selected from the group consisting of: 31; 36; 39; 42; 47; and 50.

In some specific embodiments, the amino acid sequence of the polypeptide encoded by the nucleic acid of the invention comprises, or consists of, an amino acid sequence that has a sequence identity of at least 90% with at least one sequence selected from the group consisting of: SEQ ID NO: 6; 11; 14; 17; 22; and 25.

Surprisingly, and as demonstrated herein, the combination of variations in the positions homologous to positions 424, 429 and 441 of SEQ ID NO: 1 results in a substantial increment of the thermotolerance of the enzyme.

In some embodiments of the invention, the sequence of the isolated nucleic acid of the invention comprises, or consists of, a nucleic acid sequence that has a sequence identity of at least 60% with at least one sequence selected from the group consisting of: SEQ ID NO: 26 to 42; SEQ ID NO: 45 to 50; and SEQ ID NO: 52.

In some specific embodiments of the invention, the sequence of the isolated nucleic acid of the invention comprises, or consists of, a nucleic acid sequence selected from the group consisting of: SEQ ID NO: 26 to 42; SEQ ID NO: 45 to 50; and SEQ ID NO: 52.

A second aspect of the invention relates to a polypeptide ("polypeptide of the invention") with thermotolerant laccase activity at neutral and alkaline pHs, wherein the polypeptide is encoded by the isolated nucleic acid of the invention.

In some embodiments, the amino acid sequence of the polypeptide of the invention comprises, or consists of, an amino acid sequence that has a sequence identity of at least 90% with at least one sequence selected from the group consisting of: SEQ ID NO: 1 to 17; SEQ ID NO: 20 to 25; and SEQ ID NO: 51.

In some embodiments, the amino acid sequence of the polypeptide of the invention comprises, or consists of, a sequence selected from the group consisting of: SEQ ID NO: 1 to 17; SEQ ID NO: 20 to 25; and SEQ ID NO: 51.

A third aspect of the invention relates to the polypeptide of the invention for use in the enzymatic oxidation of lignin-derived phenols. Unlike conventional laccases, the polypeptides of the invention retain their activity at neutral/alkaline pH and at high temperatures, thus being extremely useful in a number of industrial processes including, but not limited to, those listed below.

The novel tailor-made extremophilic laccases may be exploited as biocatalysts in different wood conversion applications, including those in the pulp and paper sector. In some embodiments, the polypeptides of the invention may be used in the valorisation of kraft lignins. In some embodiments, they may be used for the delignification and bleaching of kraft pulps which are carried out in bleaching sequences at alkaline pH and high temperature.

In some embodiments, the polypeptides of the invention may be used to aid fibre bonding during wood board manufacture process. The polypeptides of the invention may be also used for the formulation of lignin-based resins for wood board manufacture. In some specific embodiments, the polypeptides of the invention may be used for the formulation of resole resins for wood board manufacture.

In some specific embodiments, the polypeptides of the invention are used for the enzymatic oxidation of lignin-derived phenols. The phenols may include, but are not limited to, DMP and guaiacol. By way of example, and not limitation, the polypeptides of the invention are used for oxidation of Kraft lignins isolated from industrial black liquors.

In some specific embodiments, the enzymatic oxidation of lignin-derived phenols is performed at alkaline pHs. In some specific embodiments, the enzymatic oxidation of lignin-derived phenols is performed at pH 10 and 40ºC.

In some embodiments, the polypeptides of the invention may be used for paper pulp bleaching. In said embodiments, the use of the polypeptides of the invention (with natural phenols as mediator compounds may lead to unexpected benefits over the acidic laccases known in the state of the art (Ibarra et al. 2006. J. Chem. Technol. Biotechnol. 81:1159-1165; Camarero et al. 2007 Enzyme and Microbial Technology 40:1264-1271; Babot et al. 2011. Bioresource Technol. 102:6717- 6722), including significant improvements in pulp delignification and brightness, and in ClO₂ savings.

In some embodiments, the polypeptides of the invention may be used in the pulp and paper industry for pitch control in the so-called laccase mediator systems using phenolic compounds as redox mediators (Gutiérrez et al. 2007 Environ Sci Technol 41:4124-4129).

In some embodiments, the polypeptides of the invention may be used in the wood panel industry for the treatment of wood chips before defibrering to obtain energy savings during refining and better adhesion between the fibres.

In some embodiments, the polypeptides of the invention may be used in the wood panel industry for the formulation of lignin-based phenol-formaldehyde resins to increase lignin's reactivity and potentially reduce formaldehyde content.

In some embodiments, the polypeptides of the invention may be used for hair dyeing using phenolic compounds as natural precursors for the synthesis of dyes to replace toxic synthetic dyes such as p-phenylendiamine. The use of the polypeptides of the invention at pH 8-9 is of particular interest because alkaline pH (e.g., 8.5) helps hair swelling and dye penetration into the hair.

In some embodiments, the polypeptides of the invention may be used in the textile industry. In said embodiments, the enzymes of invention may be used for *in situ* synthesis and fixation of organic dyes in fabrics. The use of the polypeptides of the invention at an alkaline pH offers significant benefits compared to the use of acid fungal laccases (Salas et al. 2019 Green Chemistry), as most of the fiber reactive dyes will not get fixed into the cellulosic fiber (i.e., cotton) at an acidic pH.

In some embodiments, the polypeptides of the invention may be used in the textile industry to remove colour from textile wastewaters. Wastewater treatments are generally performed at high temperature and neutral/alkaline pH, conditions which are not favourable for conventional fungal laccases.

A fourth aspect of the invention relates to a host cell comprising the isolated nucleic acid of the invention ("host cell of the invention"). The present invention also encompasses a vector comprising the isolated nucleic acid of the invention.

For recombinant production of the novel laccases, the isolated nucleic acid of the invention may be inserted into a replicable vector for further cloning (amplification of the DNA) or for expression. Manipulation of the polynucleotide prior to its insertion into a vector may be desirable or necessary depending on the expression vector. The techniques for modifying polynucleotides are well known in the art.

The term "vector" as used herein refers to any nucleic acid molecule capable of transporting one or more other nucleic acid sequence(s), like the nucleotide sequence of the invention, that codifies for the polypeptide of the invention, to which it has been linked or which was introduced into said vector.

Certain vectors are capable of directing the expression of genes to which they are operatively linked. Such vectors are referred to herein as "recombinant expression vectors" or simply "expression vectors". In general, expression vectors of utility in recombinant DNA techniques are often in the form of plasmids. In the present specification, "plasmid" and "vector" may be used interchangeably, as the plasmid is the most commonly used form of vector. However, the disclosure is intended to include other forms of expression vectors, such as viral vectors (e.g., replication defective retroviruses, adenoviruses and adeno-associated viruses), which serve equivalent functions. The vector may also contain additional sequences, such as a "regulatory nucleotide sequence", a "polylinker" or "multiple cloning site" for subcloning of additional nucleic acid sequences. A "vector backbone" or "plasmid backbone" refers to a piece of DNA containing at least a plasmid origin of replication and a selectable marker which allows for selection of hosts cells containing the plasmid or vector. When a particular plasmid or vector is modified to contain non-plasmid elements (e.g., insertion of Ad sequences and/or a eukaryotic gene of interest linked to a ribosomal promoter), the plasmid sequences are referred to as the plasmid backbone. The term "promoter" as used herein refers to a nucleotide sequence required for transcription at significant levels of a second polynucleotide to which it is operably linked.

The term "regulatory nucleotide sequence" as used herein refers to a region or sequence determinants located upstream or downstream from the start of transcription and which are involved in recognition and binding of RNA polymerase and other proteins to initiate transcription. The term "regulatory nucleotide sequence" is intended to include promoters, enhancers, internal ribosomal entry sites (IRES), and other expression control elements (e.g., transcription termination signals, such as polyadenylation signals and poly-U sequences). Regulatory elements include those that direct constitutive expression of a nucleotide sequence in many types of host cell and those that direct expression of the nucleotide sequence only in certain host cells (e.g., tissue-specific regulatory sequences). In a preferred embodiment of the use of the invention the vector comprises a regulatory nucleotide sequence, preferably a promoter, operatively linked to a nucleotide sequence that codifies for the polypeptide of the invention.

The vector components include, but are not limited to, one or more of the following: a signal sequence, an origin of replication, one or more marker genes, an enhancer element, a promoter, and a transcription termination sequence.

The term "operatively linked" as used herein refers to that separate nucleotide sequences are functionally associated such that an event at one can precipitate a response from the other. Two or more operably linked nucleotide sequences can, in combination, comprise an independent genetic element, such as a recombinant expression cassette.

The vector of the invention can be comprised by a host cell ("host cell of the invention") comprising the vector of the invention, the polypeptide of the invention or the nucleotide sequence of the invention.

As used herein, the term "host cell" means any cell type that is susceptible to transformation, transfection, transduction, or the like with a nucleic acid construct or expression vector comprising the polynucleotide of the present invention. The term "host cell" encompasses any progeny of a parent cell that is not identical to the parent cell due to mutations that occur during replication. Because certain modifications may occur in succeeding generations due to either mutation or environmental influences, such progeny may not, in fact, be identical to the parent cell, but are still included within the scope of the term "host cell" as used herein. A host cell can be used, for example, for expression of a nucleic acid of interest, propagation of plasmid vectors and/or delivery of a sequence of interest to cells, wherein the nucleic acid of interest or plasmid vector are present episomally in these cells. The episomal vectors are not integrated into the host genome and are retained in self-replicating form in the host cell. The term episomally replicating is understood here as that the vector is not integrated to the genome of the host cell, but exists in parallel, is also replicated during the cell cycle and in the course of this the vector copies-depending on the number of the copies present before and after cell division-are distributed statistically in the resulting cells.

Suitable host cells for cloning or expressing the isolated nucleic acid in the vectors of the invention include, but are not limited to, prokaryote, yeast, or higher eukaryote cells.

Suitable prokaryotes include, without limitation, Gram-negative or Gram-positive bacteria, for example, *Enterobacteriaceae* such as *Escherichia,* e.g., *E. coli.* In addition to prokaryotes, eukaryotic microbes such as filamentous fungi such as *Aspergillus oryzae* or yeast are suitable cloning or expression hosts for any laccase-encoding vectors. Suitable examples of yeast host cells include, but are not limited to, *Saccharomyces cerevisiae* and *Pichia pastoris.* Importantly, *P. pastoris* (currently *Komagataella phaffii*) presents higher heterologous protein expression levels than *S*. *cerevisiae* and it is therefore particularly suitable for upscale industrial production of enzymes

In some embodiments of the invention, the host cell is an *E. coli cell.*

In some embodiments of the invention, the host cell is an *E. coli* DH5α cell.

In some embodiments of the invention, the host cell is a *Saccharomyces cerevisiae* cell.

In some embodiments of the invention, the host cell is a *Saccharomyces cerevisiae* BJ5465 cell.

In some embodiments of the invention, the host cell is a *Pichia pastoris* cell.

In some specific embodiments of the invention, the host cell is a *Pichia pastoris* X33 cell.

As stated above, the polypeptide of the invention may comprise a signal peptide linked to the N-terminal end useful to direct the polypeptide into the cell's secretory pathway when it is produced. Thus, in a preferred embodiment, the polypeptide of the invention further comprises a signal sequence at the N-terminal end. Any signal peptide can be used in the context of the present invention.

In some embodiments, the polypeptide of the invention comprises an improved signal peptide obtained from the secretion signal of the mating alpha-factor of S. *cerevisiae* through enzyme directed evolution (De Salas et al. Green Chemistry 2019, 21, 5374-5385). Said signal peptide is linked to the N-terminus of the polypeptide of the invention.

In some embodiments, the polypeptide of the invention comprises an engineered signal peptide optimized from the secretion signal of the mating alpha-factor of S. *cerevisiae* (Aza et al. 2021 CMLS 78, 3691-3707). Said signal peptide is linked to the N-terminus of the polypeptide of the invention.

In some embodiments, the signal peptide comprises an amino acid sequence having an identity of at least 90, 91, 92, 93, 94, 95, 96, 97, 98 or 99% with the sequence SEQ ID NO: 18. In some specific embodiments, the signal peptide comprises, or consists of, SEQ ID NO: 18.

In some embodiments, the signal peptide comprises an amino acid sequence having an identity of at least 90, 91, 92, 93, 94, 95, 96, 97, 98 or 99% with sequence SEQ ID NO: 19. In some specific embodiments, the signal peptide comprises, or consists of, SEQ ID NO: 19.

Additionally, plant, insect or vertebrate host cells may be used.

The host cells of the invention are transformed with the expression or cloning vectors for laccase production and cultured in conventional nutrient media modified as appropriate for inducing promoters, selecting transformants, or amplifying the genes encoding the desired sequences.

The polypeptides of the invention may be expressed intracellularly, in the periplasmic space, or directly secreted into the medium. They may be purified by any of the standard protein purification techniques known to the skilled person, including, but not limited to, anion or cation exchange chromatography, size exclusion chromatography, affinity chromatography, etc.

In some embodiments, the polypeptides of the invention are purified by anion exchange chromatography and size exclusion chromatography.

A fifth aspect of the invention relates to the host cell of the invention for use in the enzymatic oxidation of lignin-derived phenols.

All the preferred embodiments and explanations of the terms used in the fifth aspect of the invention have been explained above. All particular terms, definitions and embodiments of the third aspect of the invention are applicable to the use of the host cell of the invention for the enzymatic oxidation of lignin-derived phenols and any other phenolic compound.

Throughout the description and claims the word "comprise" and its variants are not intended to exclude other technical characteristics, additives, components or steps.

### BRIEF DESCRIPTION OF THE FIGURES

**Figure 1****.** Activities with 2,6-dimethoxyphenol (DMP) at pH 6 (light grey) and pH 8 (dark grey) of the departure point (RY2 laccase) and several of the new alkaliphilic laccases (L1-L4) subject of this invention.
**Figure 2****.** Successive shifting of optimal pH for RY2 (black circles), L1 (white diamonds), L2 (white circles), L3 (black inverted triangles), L4 (white triangles) and L5 (black squares) laccases for the oxidation of (A) 2,6-dimethoxyphenol (DMP); and (B) guaiacol through directed evolution of RY2 laccase.
**Figure 3****.** Stability of L2 (white circles), L3 (black inverted triangles) and L4 (white triangles) novel laccases at pH 10 (A) and pH 10.5 (B), room temperature.
**Figure 4****.** pH-dependent activity profile of RY2 parent laccase (black circles) and variants with mutations F396I and F392N (L1, white diamonds); mutation F396I (black squares); and mutations F396I and F392T (inverted triangles).
**Figure 5****.** pH-dependent activity profile of L1 enzyme (diamonds) and its next evolved variant L2, mutated in position 460 (F460M, circles).
**Figure 6****.** Improvement of activity with DMP at pH 8 in variant L3 (D263V), and in mutant D263G and mutant D263A, obtained from the mutagenesis of D263 in L2. Dash line indicates parental activity.
**Figure 7. Figure 7****.** Effect of the mutagenesis in positions 456-458 on the laccase pH-dependent activity profile. Shown are two examples of laccase mutants: the double L456M-A458T mutant (black circles) and the single A458L mutant (white circles), compared with parent laccase (L456 and A458; black inverted triangles).

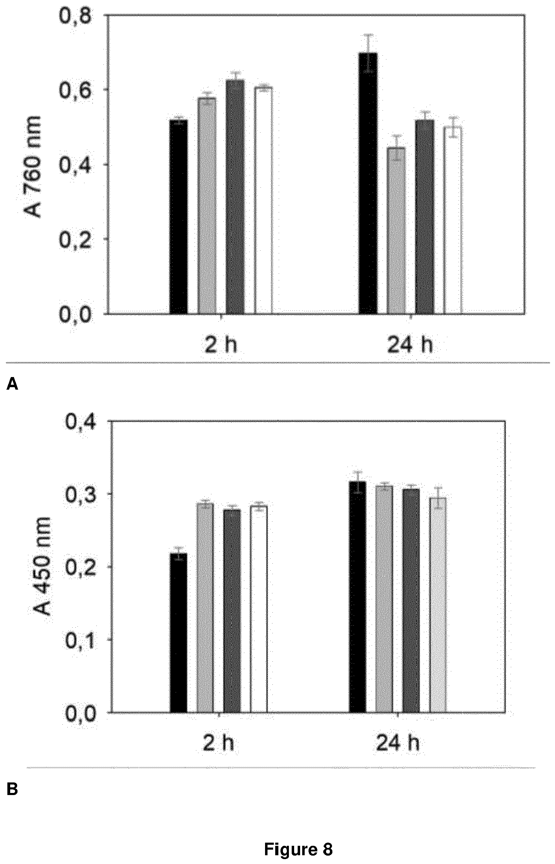

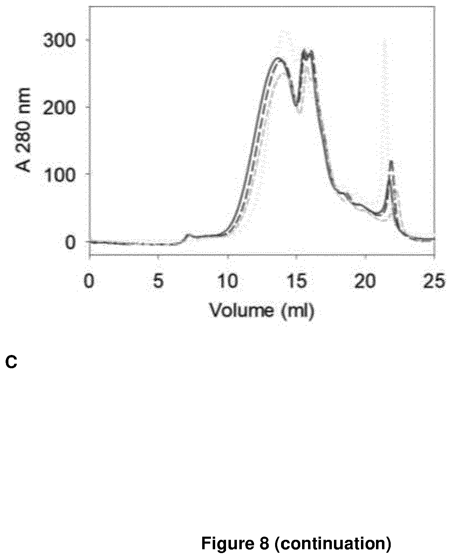

**Figure 8****.** Phenolic (A) and carbonylic (B) content of control (black) and treated kraft lignin with L2 (grey), L3 (dark grey) and L4 (white). SEC profile of kraft lignin before (dotted line) and after treatment with L2 (solid black line), L3 (dark grey dash line) and L4 (light grey dash line) laccases at pH 10 (C). Laccases clearly modify lignin's functional groups and MW, with an interesting increase of low MW lignin fractions with L4.

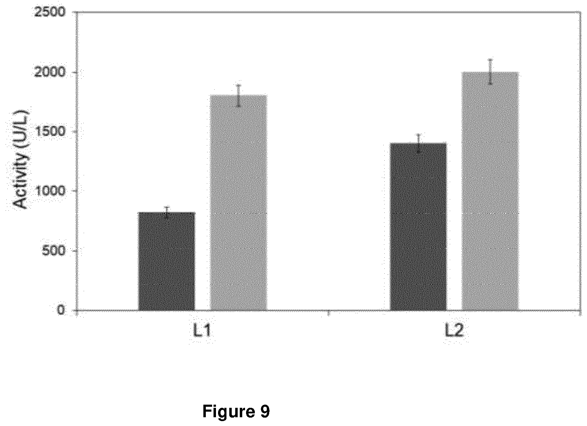

**Figure 9****.** Enzymatic activities of L1 and L2 laccases expressed in liquid cultures of P. pastoris (dark grey) and S. cerevisiae (light grey) in flasks.

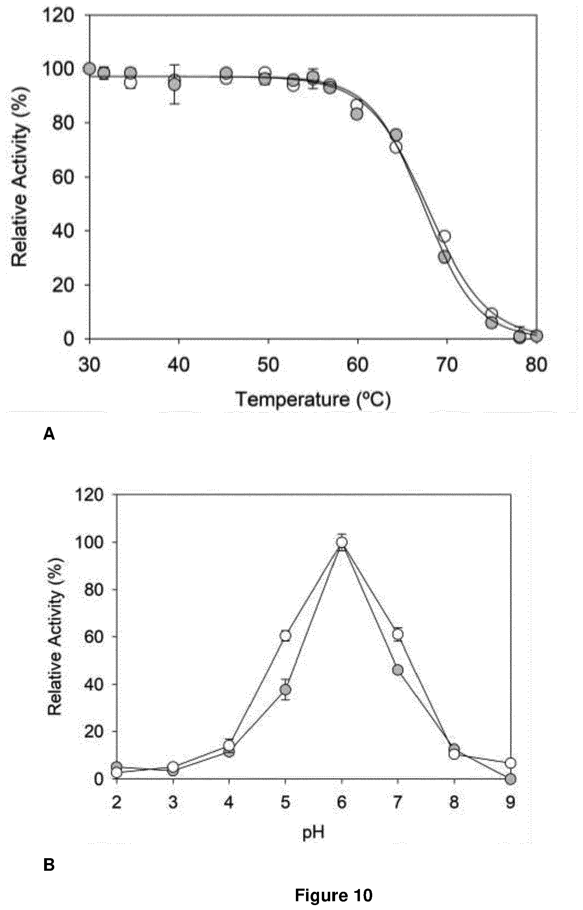

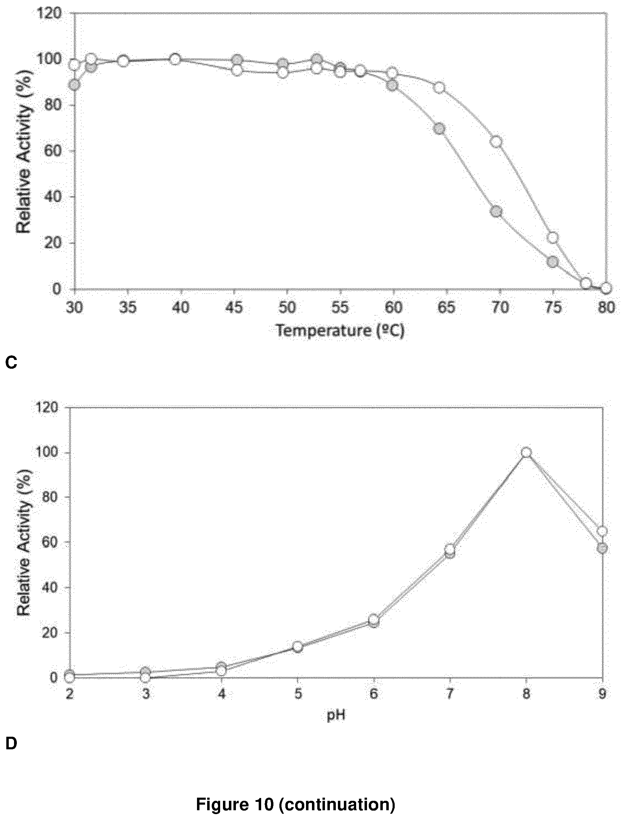

**Figure 10****.** Thermal-tolerance as T50 curves (A, B) and optimum pH (C, D) of L1 and L2 laccases expressed by P. pastoris (grey circles) or S. cerevisiae (white circles).

### EXAMPLES

### Materials and Methods

**Construction of mutant libraries.** Mutant libraries were obtained by error prone PCR and IVOE (Alcalde, M. Methods in Molecular Biology, 2010 pp 3-14), using the pJRoC30 plasmid containing laccase coding sequence fused to its evolved alpha-factor pre-proleader as template. RY2 laccase was used as parental enzyme (De Salas et al. Green Chemistry 2019, 21, 5374-5385) in the directed evolution rounds, and the best laccase mutant of each evolution round were used as parent for the next round. PCR conditions, product purification, in vivo cloning in competent cells of protease-deficient S. cerevisiae BJ5465 strain and HTS microplate fermentations have been described previously (Pardo et al. Cell. Mol. Life Sci. 2015 72:897-910).

**Screening of mutant libraries.** For epPCR library, activities of 2800 *S*. *cerevisiae* clones expressing laccase mutants were screened. For Saturation Mutagenesis activities of at least 145 mutant colonies of *S*. *cerevisiae* expressing laccase were screened with ABTS and lignin-derived phenols (99% coverage of all possible substitutions) along with their parental laccase. In the case of Combinatorial Saturation Mutagenesis of residues 456-458, activities of 3450 mutant colonies of the resultant CSM library were screened (95% coverage of NTS VMS VNA possible substitutions). The 96-well plates were centrifuged (6000rpm, 4º C, 5min) and 20µL supernatant from each micro-fermentation were transferred to replica 96-well plates using a liquid-handling robot (Quadra96, Tomtec, USA). Enzymatic reactions were started by adding 180µL substrate solution (3mM ABTS in 50mM Britton-Robinson buffer pH 3, 3mM DMP in 50mM Britton-Robinson buffer pH 6 and 8, and 9mM guaiacol in 50mM Britton-Robinson buffer pH 9). Laccase activity in the wells were measured in a Spectra max plus 384 plate reader (Molecular Devices) by monitoring the oxidation of the substrate at the maximum absorbance of the oxidized product (ABTS: 418nm, DMP: 469nm, guaiacol: 470nm) in kinetic mode.

**Flask production and purification.** The *S*. *cerevisiae* clones expressing selected laccase variants were grown in duplicate, in 100mL flasks with 30mL expression medium (containing 1M CuSO4 and 3% ethanol) (Camarero et al. Applied and Environmental Microbiology 2012, 78,5). Laccase activity secreted in the liquid cultures was monitored over time by measuring the oxidation of 3mM ABTS in 100mM Britton-Robinson buffer pH 3, 3mM DMP 100mM Britton-Robinson Buffer (pH 6 and 8) and 9mM guaiacol in 100mM Britton-Robinson Buffer pH 9 with UV-1900 Shimadzu spectrophotometer. After 3 days of fermentation, cultures were treated as previously described (De Salas et al. Green Chemistry 2019 21, 5374-5385). Purification of the enzymes by High Pressure Liquid Chromatography (HPLC) was carried out in 3 chromatographic steps: two anion exchange steps using a HiPrep-QFF 16/10 column in a 100mL gradient of 0-40% elution buffer, and a MonoQ-HR 5/5 column in a 30mL gradient of 0-25% elution buffer, followed by size exclusion chromatography with a Superdex75. Fractions containing laccase activity (with 3mM ABTS in 50mM citrate-phosphate pH 3) were pooled, dialyzed in Tris-HCI pH 7 and concentrated after each chromatographic step. Enzyme purification was confirmed by the electrophoretic mobility in SDS-PAGE (12% acrylamide) stained with Coomassie brilliant blue.

**Enzyme characterisation.** Characterisation assays with pure and non-purified (crude) laccases were carried out in 96-well plates with enzyme aliquots of 0.1U/mL activity (measured with 3mM ABTS in 100mM B&R buffer at the corresponding optimum pH for each enzyme). Laccase activities were monitored spectrophotometrically in the plate reader by measuring the increase of absorbance of the oxidised products at room temperature.

Optimum pH: reactions of 20µL enzyme with 180µL 3mM ABTS, 3mM DMP or 9 mM guaiacol solutions (in 100mM B&R buffer pH 2-10) were carried out by triplicate and the laccase activities monitored in kinetic mode. The relative activities were calculated as a percentage of the maximum activity obtained for each laccase variant.

T50 (10 min): 35µL enzyme aliquots were transferred to 96-well PCR plates, sealed and incubated at a temperature gradient of 30 to 80 ºC during 10min in a thermocycler (two assays with temperature ramps of 30-55 ºC and 55-80ºC were performed). Then, plates were cooled on ice for 10min and tempered for 5 min. Then, 20 µL samples were transferred to 96-well plates with 180µL 3mM ABTS in 50mM citrate-phosphate buffer (pH 3), and laccase activities were measured.

Medium term pH-stability assay: Enzymes were incubated for 1h at 20 ºC with 20 mM Tris-HCI buffer at pH 10-10.5. Then, 20 µL-samples taken at different incubation times were added to 96-well plates filled with 180 µL 3mM ABTS in 50mM citrate-phosphate buffer pH 3 to measure laccase activity.

Kinetic constants for the oxidation of ABTS (418=36000M-1cm-1) and DMP (469=27500M-1cm-1) were measured in triplicate with 20 µL of pure enzyme aliquots added to 230µL solutions of 3mM ABTS in 50 mM citrate-phosphate pH 3, 3mM DMP in 100 mM sodium-phosphate pH 8, 9 or 10 mM. To calculate Km and kcat values the average Vmax was represented versus substrate concentration and fitted to a single rectangular hyperbola function in SigmaPlot (version 14.0) software, where parameter "a" was equal to kcat and parameter "b" was equal to Km.

**Heterologous expression in *Pichia pastoris.*** Expression in *Pichia pastoris* was carried out following manufacturer's instructions (Invitrogen). First, laccase CDS was amplified by PCR with "BstBI long target" and "Ext pJRoc-R" primers. Then, purification of the PCR product was digested with Notl and BstBI to integrate the laccase in the pPICZ-B vector (both, laccase and vector present those restriction sites). Linearized pPICZ-B vector with zeocin resistance was ligated with the digested laccase gene by using T4 DNA ligase, in proportion 1:3 (50ng of vector and 37.5ng of laccase) and left overnight at 16ºC.

Ligation product was transformed in *E. coli* and incubated in LB-zeocin plates at 37ºC overnight. Plasmids were isolated with High Pure Plasmid Isolation Kit and digested with Sacl in a specific region integrated in AOX (methanol promoter) to linearize the construction and integrate in the genome of *P. pastoris.*

Transformation of *P. pastoris* X33 strain was performed following the transformation method provided by the manufacturer. Transformed cells were plated in YPD-zeocin and incubated for 2-4 days at 28ºC. The obtained colonies were transferred to BMM ABTS agar plates and incubated for 2-4 days at 28ºC until formation of a green halo (due to oxidation of ABTS) indicated the presence of laccase activity.

### Results

Laccase RY2 (De Salas et al. Green Chemistry 2019 21, 5374-5385) was used as the departure point for the enzyme engineering work. The new extremophilic enzymes described herewith differ from RY2 in up to 9 residues that confer novel enzymatic properties to the variants (positions homologous to positions 392, 396, 460, 263, 456, 458, 424, 429, and 441 of SEQ ID NO:1).

RY2 laccase mature sequence (not including the signal peptide) differs in twelve mutations from Chu-B laccase (ES2525195 B1).

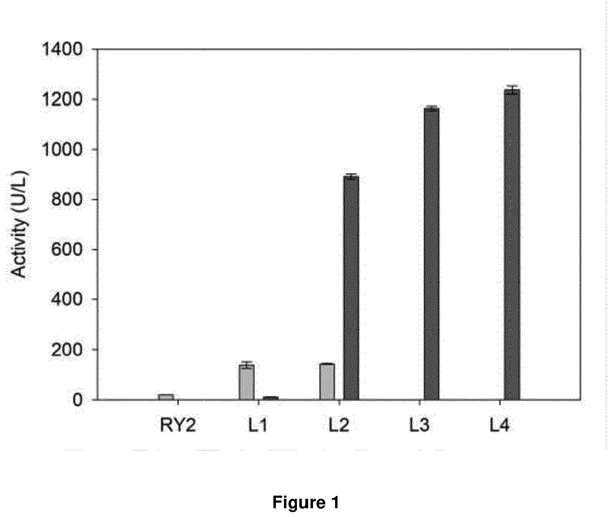

**Enzymatic oxidation of lignin-derived phenols.** The optimum pH for the enzymatic oxidation of lignin-derived phenols by RY2 is acidic. The parental laccase displays a extremely poor initial activity at pH 6 with 2, 6-dimethoxyphenol (DMP) as a substrate, and no detectable activity at pH >6 (Fig. 1). By contrast, the new, evolved variants show high activity at pH 8 with the same substrate (Fig. 1).

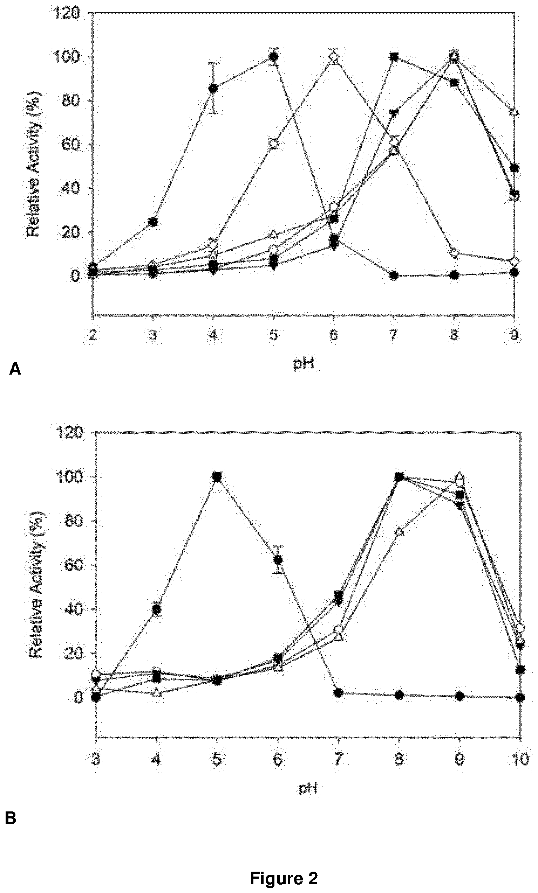

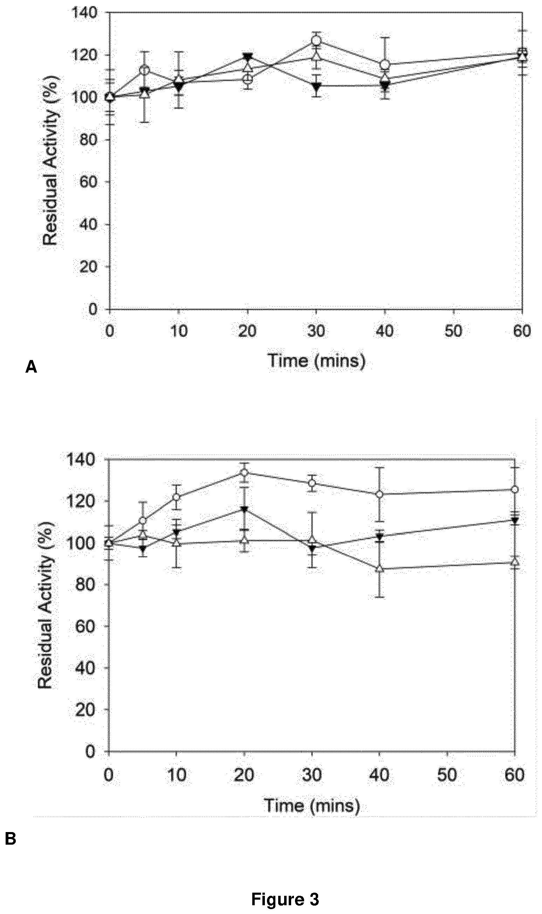

Indeed, the directed evolution of the parental laccase sequence enabled a notable shift of the optimum pH for the enzymatic oxidation of phenols, e.g., DMP and guaiacol from acidic pH values (RY2) to pH 8 (Fig. 2A) and pH 9 (Fig. 2B), respectively. Moreover, the novel alkaliphilic laccase variants are stable up to pH 10-10.5 (Fig. 3). All these variants are thermotolerant, with T50 values from 73ºC to 80ºC (Table 1).

**Table 1. T50 (10 min) values of the different extremophilic laccases. T50 represents the temperature at which the enzyme retains 50% of activity activity after a certain incubation period (10 min in this case).**

| **Laccase** | **T50** |
|---|---|
| RY2 | 74 |
| L1 (+F392N) | 75 |
| L2 (+F460M) | 73 |
| L3 (+D263V) | 73 |
| L4 (+A458L) | 73 |
| L5 (+V424T+S429T+D441N) | 80 |
| L4R (A461T) | 69 |

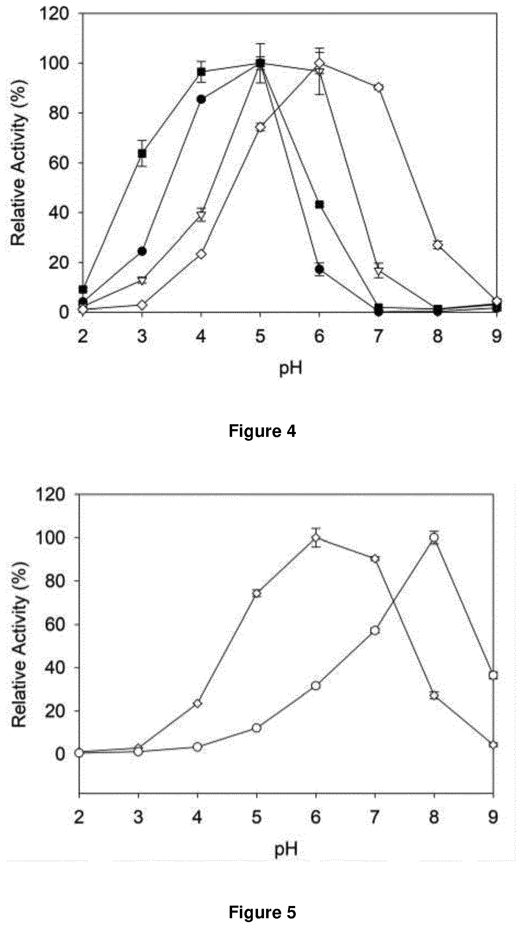

Substitution of Phe 392 by Asn or Thr clearly shifted the maximum laccase activity towards more neutral pH values. Mutation F392T shifted the optimum pH with DMP from pH 5 to pH 6. The effect of mutation F392N (in L1 variant) was even more pronounced, significantly enhancing the activity at pH 7 and even pH 8. (Fig. 4). Since the two variants mutated in 392 also held mutation F396I, that was jointly selected in the same evolution round, and in order to demonstrate the positive impact of mutations in 392 to improve the activity at neutral pH, a laccase variant solely mutated in F396I was added for comparison with the double mutated variant L1 (F392N, F396I) and variant F392T, F396I (Fig 4). Since the presence of Asn or Thr in 392 position is essential for conferring laccase activity at neutral pH, it is expected that substitution of Phe 392 by other amino acids of the same group, that is, with polar uncharged side-chains like Ser or Gin, will cause similar effects as those proved within the present invention.

Mutation F460M also produces a clear shift in the optimum pH of the reaction, from pH 6 to pH 8 (DMP). Importantly, the mutation enables catalysis at pH 9 (Fig 5). Indeed, these are the first reported kinetic parameters of the enzyme at pH 9 (Table 2). Therefore, this mutation is considered crucial for the alkalophilicity of laccase activity.

**Table 2. Kinetic constants for the oxidation of ABTS (reference activity) and of lignin derived phenol (DMP) by the novel alkaliphilic laccases obtained through evolution of RY2 laccase.**

| **Clone** | **Substrate** | **pH** | **k_{cat} (s⁻¹)** | **Kₘ (mM)** | **k_{cat} /Kₘ (mM⁻¹ s⁻¹)** |
|---|---|---|---|---|---|
| RY2 | ABTS | 3 | 543 ± 17 | 0.01 ± 0.001 | 52240 ± 569 |
| | DMP | 5 | 123 ± 6 | 0.26 ± 0.04 | 479 ± 76 |
| L2 | ABTS | 3 | 231 ± 5.6 | 1.29 ± 0.11 | 179 ± 16 |
| | DMP | 8 | 89 ± 6.2 | 20 ± 2.4 | 4 ± 0.6 |
| | DMP | 9 | 9.5 ± 0.1 | 1.1 ± 0.03 | 9 ± 0.1 |
| L3 | ABTS | 3 | 123 ± 5.5 | 0.46 ± 0.04 | 266 ± 26 |
| | DMP | 8 | 82.3 ± 7.6 | 1.5 ± 0.12 | 54.9 ± 6.7 |
| | DMP | 9 | 21.7 ± 1.3 | 0.3 ± 0.03 | 72 ± 8.47 |
| | DMP | 10 | 0.79 ± 0.03 | 0.027 ± 0.001 | 29 ±1.5 |
| L4 | ABTS | 3 | 136 ± 2.7 | 0.68 ± 0.04 | 200 ± 12 |
| | DMP | 8 | 42 ± 1.26 | 1.71 ± 0.1 | 25 ± 1.6 |
| | DMP | 9 | 26 ± 0.28 | 0.43 ± 0.01 | 61 ± 1.6 |
| | DMP | 10 | 2 ± 0.05 | 0.022 ± 0.002 | 95 ± 9 |

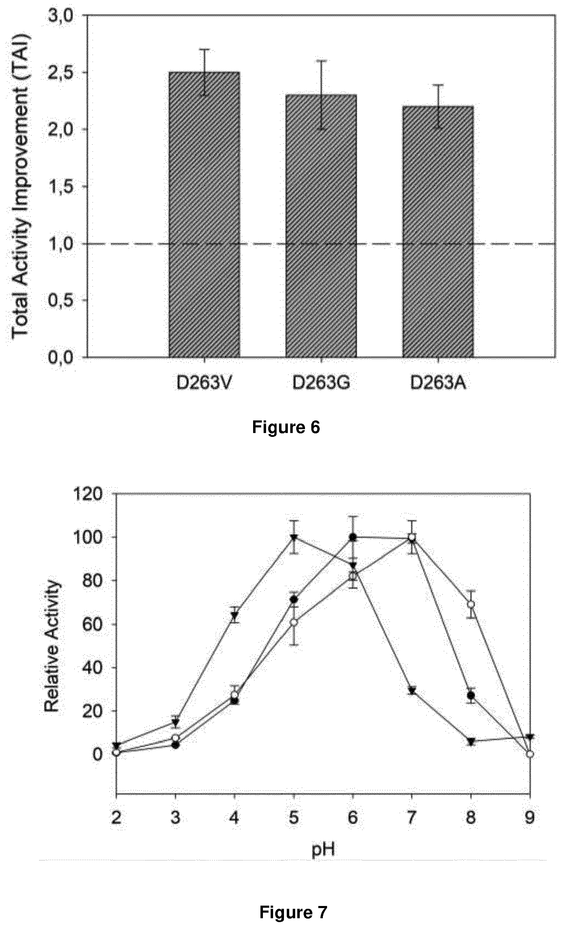

Mutation D263V notably improves (around 2.5-fold) the catalytic efficiency of the enzyme at pH 8-9 in L3 and even allows to measure the kinetic constants of the enzyme at pH 10 (Table 2). The same effect was observed when Asp263 was substituted by other non-polar and small amino acids, such as Ala or Gly (Fig. 6). Thus, replacement of Asp263 by non-polar and small amino acids is crucial for the alkalophilicity of laccase activity.

Mutation of the positions 456-458 also has a strong effect in the alkaliphilic activity of the enzyme. Some available examples are mutation A458L (present in variant L4) and the double mutant L456M, A458T, both of them producing a remarkable shift of the optimum pH (Fig. 7). Besides, mutation A458L produces a notable increase in laccase catalytic efficiency for the oxidation of DMP at pH 10 in L4 (Table 2).

## Claims

1. An isolated nucleic acid encoding a polypeptide with thermotolerant laccase activity at neutral and alkaline pHs, wherein the amino acid sequence of the polypeptide encoded by the isolated nucleic acid:
(i) has a sequence identity of at least 90% with SEQ ID NO: 1; and
(ii) comprises (a) an amino acid mutation in the position homologous to position 392 of SEQ ID NO:1; and (b) an isoleucine residue in the position homologous to position 396 of SEQ ID NO: 1.

2. The isolated nucleic acid of claim 1, wherein, in the amino acid sequence of the polypeptide encoded by the isolated nucleic acid, the amino acid in the position homologous to position 392 of SEQ ID NO: 1 is substituted by an amino acid selected from the group consisting of: serine; glutamine; asparagine; and threonine.

3. The isolated nucleic acid of any one of claims 1 to 2, wherein the amino acid sequence of the polypeptide encoded by the isolated nucleic acid further comprises an amino acid mutation in the position homologous to position 460 of SEQ ID NO:1.

4. The isolated nucleic acid of any one of claims 1 to 3, wherein the amino acid sequence of the polypeptide encoded by the isolated nucleic acid further comprises an amino acid mutation in the position homologous to position 263 of SEQ ID NO:1.

5. The isolated nucleic acid of any one of claims 1 to 4, wherein the amino acid sequence of the polypeptide encoded by the isolated nucleic acid further comprises an amino acid mutation in the position homologous to position 458 of SEQ ID NO:1.

6. The isolated nucleic acid of claim 5, wherein the amino acid sequence of the polypeptide encoded by the isolated nucleic acid further comprises an amino acid mutation in the position homologous to position 456 of SEQ ID NO:1.

7. The isolated nucleic acid of any one of claims 1 to 6, wherein the amino acid sequence of the polypeptide encoded by the isolated nucleic acid further comprises amino acid mutations in the position homologous to: position 424, 429 and 441 of SEQ ID NO:1.

8. The isolated nucleic acid of any one of claims 1 to 7, wherein the sequence of the isolated nucleic acid comprises, or consists of, a nucleic acid sequence that has a sequence identity of at least 60% with at least one sequence selected from the group consisting of: SEQ ID NO: 26 to 42; SEQ ID NO: 45 to 50; and SEQ ID NO: 52.

9. The isolated nucleic acid of any one of claims 1 to 8, wherein the sequence of the isolated nucleic acid comprises, or consists of, a nucleic acid sequence selected from the group consisting of: SEQ ID NO: 26 to 42; SEQ ID NO: 45 to 50; and SEQ ID NO: 52.

10. A polypeptide with thermotolerant laccase activity at neutral and alkaline pHs, wherein the polypeptide is encoded by the isolated nucleic acid of any one of claims 1 to 9.

11. The polypeptide of claim 10, wherein the amino acid sequence of the polypeptide comprises, or consists of, an amino acid sequence that has a sequence identity of at least 90% with at least one sequence selected from the group consisting of: SEQ ID NO: 1 to 17; SEQ ID NO: 20 to 25; and SEQ ID NO: 51.

12. The polypeptide of any one of claims 10 to 11, wherein the amino acid sequence of the polypeptide comprises, or consists of, a sequence selected from the group consisting of: SEQ ID NO: 1 to 17; SEQ ID NO: 20 to 25; and SEQ ID NO: 51.

13. The polypeptide of any one of claims 10 to 12 for use in the enzymatic oxidation of lignin-derived or any other natural phenols.

14. A host cell comprising the isolated nucleic acid of any one of claims 1 to 9 and/or the polypeptide of any one of claims 10 to 12.

15. The host cell of claim 14 for use in the enzymatic oxidation of lignin-derived or any other natural phenols.
